(19) Europäisches Patentamt
European Patent Office
Office européen des brevets

(11) EP 3 786 304 A1

(12) EUROPEAN PATENT APPLICATION
published in accordance with Art. 153(4) EPC

(43) Date of publication:
**03.03.2021 Bulletin 2021/09**

(21) Application number: **19792125.7**

(22) Date of filing: **25.04.2019**

(51) Int Cl.:
***C12Q 1/02*** *(2006.01)* ***A61K 31/437*** *(2006.01)*
***A61P 35/00*** *(2006.01)*

(86) International application number:
**PCT/CN2019/084176**

(87) International publication number:
**WO 2019/206203 (31.10.2019 Gazette 2019/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.04.2018 CN 201810391658**

(71) Applicant: **Xiamen University**
**Xiamen, Fujian 361005 (CN)**

(72) Inventors:
- **WANG, Hongrui**
  **(CA)**
- **DENG, Xianming**
  **Xiamen, Fujian 361005 (CN)**
- **ZENG, Taoling**
  **Xiamen, Fujian 361005 (CN)**
- **JIANG, Tingting**
  **Xiamen, Fujian 361005 (CN)**

(74) Representative: **Elzaburu S.L.P.**
**Miguel Angel 21, 2nd floor**
**28010 Madrid (ES)**

# (54) METHOD FOR ADJUSTING RAS UBIQUITINATION

(57) Provided is use of E3 ubiquitin ligase Nedd4-1 in preparation of a drug for inhibiting the Ras protein level. Also provided is use in preparation of a drug for preventing and/or treating tumors or a drug for prolonging the life span of organisms.

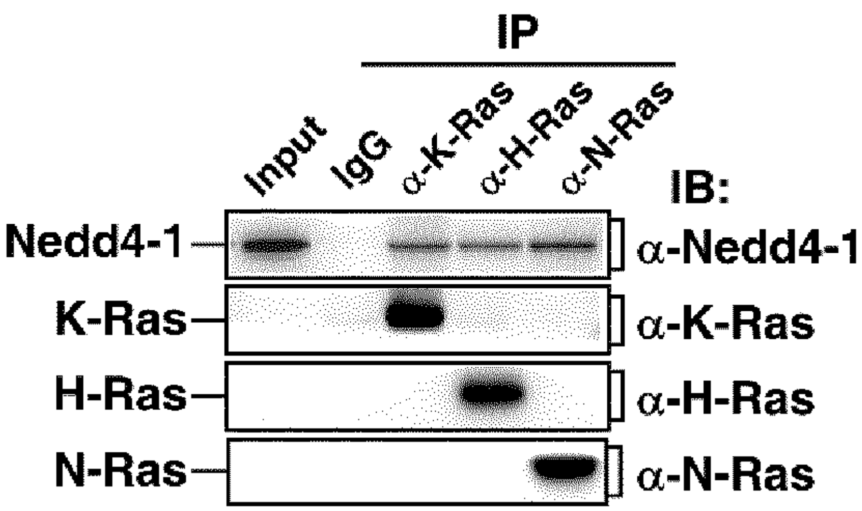


Fig. 1 (a)



Printed by Jouve, 75001 PARIS (FR)

(Cont. next page)

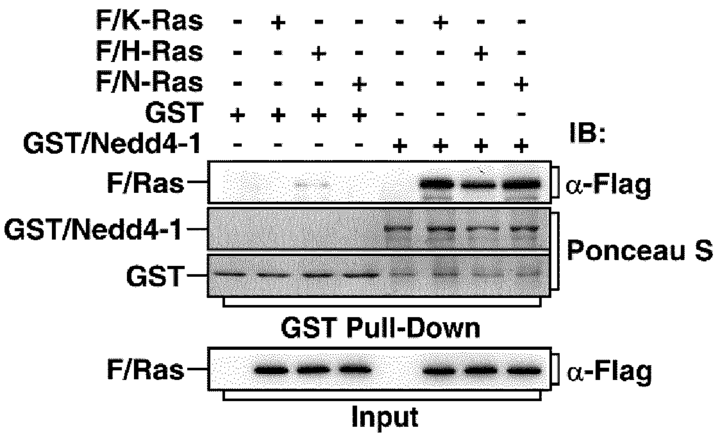
F/K-Ras - + - - - + - -
F/H-Ras - - + - - - + -
F/N-Ras - - - + - - - +
GST + + + + - - - -
GST/Nedd4-1 - - - - + + + +
IB:
F/Ras
α-Flag
GST/Nedd4-1
Ponceau S
GST
GST Pull-Down
F/Ras
α-Flag
Input

Fig. 1 (b)

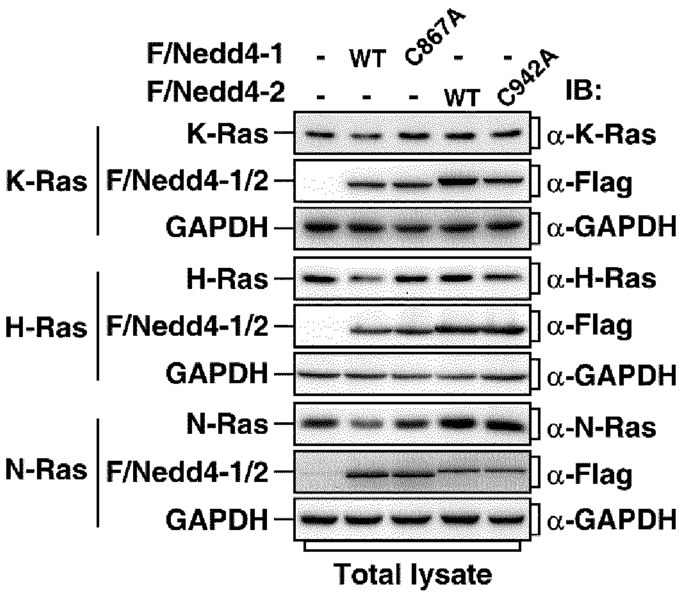
F/Nedd4-1 - WT C867A - -
F/Nedd4-2 - - - WT C942A
IB:
K-Ras
K-Ras
α-K-Ras
F/Nedd4-1/2
α-Flag
GAPDH
α-GAPDH
H-Ras
H-Ras
α-H-Ras
F/Nedd4-1/2
α-Flag
GAPDH
α-GAPDH
N-Ras
N-Ras
α-N-Ras
F/Nedd4-1/2
α-Flag
GAPDH
α-GAPDH
Total lysate

Fig. 1 (c)

## Description

### Technical Field

The present invention relates to the field of biotechnology, and specifically relates to a method of regulating ubiquitination of Ras through an E3 ubiquitin ligase Nedd4-1.

### Background Art

Ras protein, as an intracellular molecular switch, switches between an inactive state in which it binds to GDP (guanosine diphosphate) and an active state in which it binds to GTP (guanosine triphosphate), and plays a key role in many physiological and biochemical regulation processes in cells, including regulation and control in gene expression, cell growth and differentiation, cell cycle, cytoskeleton and cell migration, vesicular transport, transport between the cell nucleus and the cytoplasm, etc. GTP binding allows multiple residues of the Ras protein (primarily residues 30-40 in the switch 1 region and residues 60-70 in the switch 11 region) to form a conformation that allows the Ras effector protein to bind thereto, the conversion of the Ras protein between the active and inactive states is regulated by GAPs (GTPase activating proteins) and GEFs (guanosine monophosphate exchange factors). Ras gene mutation can result in the damage of intrinsic GAPase activity of Ras protein or preventing GAP from binding with Ras protein, thus leading to the continuous activation of downstream signaling pathways and promoting the occurrence and development of tumors. At the same time, Ras proteins (comprising H-Ras, K-Ras and N-Ras) are also the most common proto-oncoproteins in humans, and are closely related to tumor growth and transformation activity. Ras gene mutations were found in 20%-30% of all human tumors, including 90% of pancreatic cancer, 50% of colon cancer, 30% of lung cancer, 50% of thyroid cancer and 30% of myeloid leukemia. Studies have shown that many tumors depend on the continuous expression of Ras protein at the cellular level and in animal models. Therefore, the research on Ras family proteins has always been paid great attention by researchers. Nedd4-1 is an E3 ubiquitin ligase belonging to the HECT family, structurally includs an N-terminal C2 domain, an intermediate WW domain and a C-terminal HECT domain, which has an amino acid sequence shown by Accession: NP_006145 in NCBI protein database. This factor is capable of degrading Ras proteins by ubiquitination. Ubiquitination of protein molecules is an important covalent modification of intracellular proteins after translation, which regulates many important biological functions including degradation of protein molecules, DNA repair, gene transcription, cell cycle, endocytosis and so on. Protein degradation mediated by ubiquitination plays an important role in maintaining the normal biological function of cells, and abnormal protein degradation can cause many diseases including cancer. Some study results in recent years have shown that inhibition of the activity of protease complexes has some therapeutic effect on the treatment of some diseases such as cancer, autoimmune diseases, inflammation and cardiovascular diseases. At present, proteasome inhibitors such as Bortezomib have been approved by the US FDA for the treatment of clinical tumors, particularly treating malignant tumors such as multiple myeloma. However, the application of protease complex inhibitors is greatly limited because of its non-specific effect on the degradation of almost all proteins, which inevitably leads to strong toxic side effects.

### Summary of the Invention

In one aspect, the present invention relates to a method of identifying a compound that regulates Ras level, the method comprising:

a. contacting a Ras-expressing cell or an in vitro ubiquitination system with a test compound;
b. selecting a compound that can increase or decrease the Ras level of the cell or system in step a.

Optionally, the method further comprising:

c. detecting whether the compound obtained in step b regulates Ras level depending on the presence of Nedd4-1 protein or not; and
d. selecting compounds that regulate Ras level depending on the presence of the Nedd4-1 protein.

According to the foregoing method, wherein the Ras-expressing cell is a cell expressing exogenous Ras or endogenous Ras, or both.
A method according to any one of the preceding, wherein in step b a compound that can decrease the Ras level of the cell or system in step a is selected.
In another aspect, the present invention relates to the compounds obtained by screening by using the method of any one of the preceding.

According to the foregoing compounds, wherein the compounds are capable of promoting ubiquitination degradation of Ras in a Nedd4-1 protein-dependent manner (i.e., the compounds can promote Ras protein degradation in the presence of the Nedd4-1 protein, but the ability of the compounds to promote Ras protein degradation is significantly diminished in the absence of the Nedd4-1 protein).

A compound according to the preceding, is selected from:

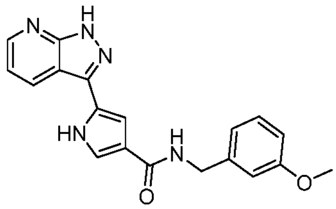

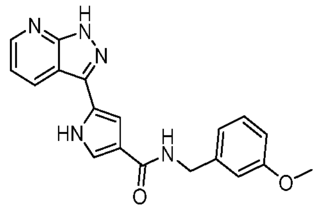

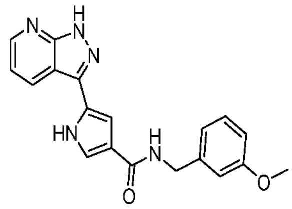

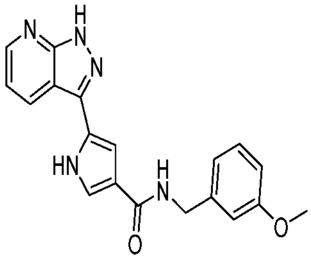

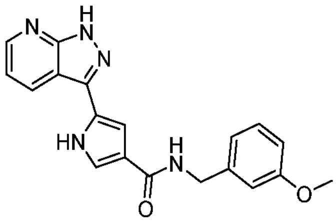

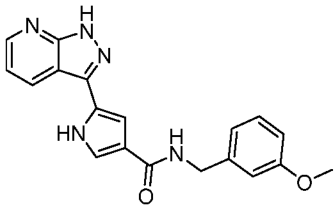

.

Preferably, the compound is selected from:

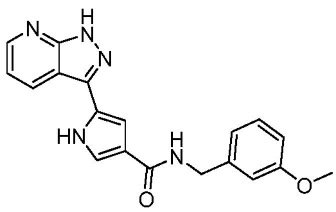

,

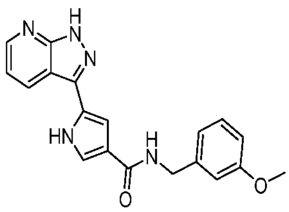

,

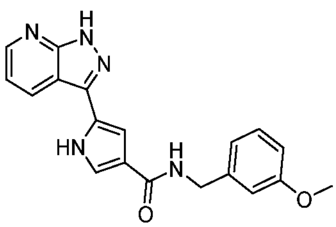

.

In one aspect, the present invention relates to a method of regulating Ras level in a cell, the method comprising:

a. contacting a compound that regulates ubiquitination degradation of Ras with a Ras-expressing cell or an in vitro ubiquitination system; and
b. optionally, the level of Nedd4-1 in the cell or system is also regulated.

According to the foregoing method, regulating the level of Nedd4-1 comprising: a. gene editing, b. overexpression, and c. RNA interference.

The method according to any one of the preceding, wherein the Ras is H-Ras, K-Ras or N-Ras and various mutants thereof.

The method according to any one of the preceding, wherein the compound that regulates ubiquitination degradation of Ras is a compound according to any one of the preceding.

The method according to any one of the preceding,, the activity of the compound to regulate ubiquitination degradation of Ras is Nedd4-1 dependent or independent.

In one aspect, the present invention relates to a method of regulating Ras level, the method comprising:

a. judging the state of Ras protein;
b. if the Ras protein is a wild-type protein or in an inactive state, regulating the Ras level by regulating the expression level of Nedd4-1;
c. if the Ras protein is a mutant protein or in an active state, regulating the Ras level by adding a compound.
d. optionally, regulating the expression level of Nedd4-1 and adding the compound can be performed simultaneously.

In one aspect, the present invention relates to a method of regulating Ras level, the method comprising:

a. judging whether Ras level can be regulated by Nedd4-1;
b. if the Ras level can be regulated by Nedd4-1, regulating the Ras level by regulating the expression level of Nedd4-1;
c. if the Ras level cannot be regulated by Nedd4-1, then Ras level is regulated by adding compounds.
d. optionally, regulating the expression level of Nedd4-1 and adding the compound can be performed simultaneously.

The method according to any one of the preceding, wherein the Ras level is decreased by up-regulating the level of Nedd4-1, or increased by down-regulating the level of Nedd4-1. The method according to any one of the preceding, wherein the level of Ras protein is decreased by adding a compound, preferably the level of Ras protein is decreased without affecting the mRNA level of Ras.

In one aspect, the present invention relates to a method of identifying a compound for preventing or treating cancer or tumor, the method comprising:

a. contacting a tumor cell line in vitro with a compound of the present invention, optionally the cancer or tumor has a Ras positive genetic background,
b. selecting a compound capable of inhibiting or decreasing the colony forming ability of the cell as a candidate drug.

In one embodiment of the present invention, if the drug to be tested is capable of inhibiting or decreasing the colony forming ability of cancer cell, it may be used as a candidate drug. A method according to any one of the preceding, which is carried out in vitro.

A method according to any one of the preceding, which is carried out in vivo.

In one aspect, the present invention relates to a method of preventing or treating cancer or tumor, the method comprising administering a compound that promotes ubiquitination degradation of Ras to a subject suffering from cancer or tumor or to a subject at high risk of having a cancer or tumor, optionally screening compounds that promote ubiquitination degradation of Ras by using the method of any one of the preceding. Optionally, the cancer or tumor has a Ras positive genetic background.

The method according to any one of the preceding, wherein the Ras is H-Ras, K-Ras or N-Ras and various active mutants thereof, including but not limited to G12V, G12D, G13V, G13D and Q61R. Preferably, the mutant is G12V, G13D and Q61R of H-Ras; G12V, G12D, G13V, G13D and Q61R of K-Ras; G12V, G13V, G13D and Q61R of N-Ras. Most preferably, the mutant is G12V of K-Ras.

In one aspect, the present invention relates to the use of a compound that regulates the expression level of Nedd4-1 in preparing a drug for the prevention or treatment of cancer or tumors. The compound that regulates the expression level of Nedd4-1 include, but are not limited to, nucleic acid constructs, small molecule compounds, antibodies, and the like.

In one aspect, the present invention relates to the use of a compound that promotes ubiquitination degradation of Ras protein in preparing a drug for the prevention or treatment of cancer or tumors, preferably the compound that promotes ubiquitination degradation of Ras protein is Nedd4-1 dependent, optionally the compound is a compound according to any one of the preceding.

In one aspect, the present invention relates to the use of a compound that promotes ubiquitination degradation of Ras proteins in preparing a drug for the prevention or treatment of Ras-associated autoimmune diseases, inflammations, and cardiovascular diseases.

## Brief Description of the Drawings

Fig. 1 shows that Nedd4-1 regulates Ras protein levels. Fig. 1(a) Interaction of endogenous Nedd4-1 with Ras proteins in HeLa cells. Fig. 1(b) Direct interaction of Nedd4-1 protein with Ras protein in vitro. Fig. 1(c) Overexpression of Nedd4-1 protein can decrease the level of endogenous Ras protein in HeLa cells. The statistical result is the mean ± SD of three independent experiments. Fig. 1(d) Overexpression of Nedd4-1 protein can decrease exogenous Ras protein level. Fig. 1(e) Nedd4-1-mediated degradation of Ras proteins is through the lysosomal pathway. Fig. 1(f) Nedd4-1 mediates in vivo ubiquitination of H-Ras and N-Ras. Fig. 1(g) H-Ras and N-Ras were directly modified by ubiquitination with Nedd4-1 under in vitro conditions. Fig. 1(h) Nedd4-1 regulates the half-life of H-Ras proteins. Fig. 1(i) Nedd4-1 regulates the half-life of N-Ras proteins. Fig. 1(j) Overexpression of Nedd4-1 in HT-29 cells can decrease endogenous WT K-Ras protein level, and knocking down Nedd4-1 can upregulate endogenous WT K-Ras protein level. Fig. 1(k) K-Ras G12V mutant did not significantly degrade when F/Nedd4-1 was overexpressed. Fig. 1(l) K-Ras G12V mutation attenuates Nedd4-1-mediated K-Ras ubiquitination in vivo. Fig. 1(m) K-Ras G12V mutation attenuates Nedd4-1-mediated K-Ras ubiquitination in vitro.

Fig. 2 shows the screening of compounds that can decrease the level of exogenous K-Ras-G12V protein. Fig. 2(a) 28 compounds were screened by dot-blotting high-throughput screening, which overexpressed Flag-K-Ras-G12V and Flag-Nedd4-1-WT in 293T cells, and 7 compounds that can decrease the level of exogenous K-Ras-G12V protein were screened by dosing treatment. Fig. 2(b) 7 compounds that can decrease exogenous K-Ras-G12V were further screened in SW620 cells and 3 compounds that can decrease the level of endogenous K-Ras-G12V protein were selected. Fig. 2(c) 3 compounds after endogenous and exogenous screening, it was tested whether the decrease of K-Ras-G12V protein levels by the 3 compounds was dependent on the presence of Nedd4-1 protein.

Fig. 3 shows that the compounds obtained by screening have a dose-effect relationship for decreasing endogenous K-Ras-G12V protein levels in cells. Fig. 3(a) Compound ZT-10-158-01 was effective in decreasing the level of endogenous K-Ras-G12V protein in SW620 cells with a positive concentration-dependent inhibitory effect. Fig. 3(b) Compound ZT-10-158-01 was effective in decreasing the level of exogenous K-Ras-G12V protein in HEK293T cells with a positive concentration-dependent inhibitory effect.

Fig. 4 shows that compounds obtained by screening selectively inhibit colony formation of tumor cells with Ras signaling disorders.

Fig. 4(a) Compound ZT-10-158-01 was effective in inhibiting colony forming ability of MCF-7 cells on agar.

Fig. 4(b) Compound ZT-10-158-01 was effective in inhibiting colony forming ability of SW620 cells on agar.

Fig. 4(c) Compound ZT-10-158-01 did not inhibit the colony forming ability of HT-29 cells on agar.

## Detailed Description of the Invention

The following are examples provided to assist those skilled in the art in practicing the present invention. Modifications and variations may be made in the embodiments described herein by those of ordinary skill in the art without departing from the spirit or scope of the present disclosure. All publications, patent applications, patents, Figs and other references mentioned herein are expressly incorporated by reference in their entirety.

Unless defined otherwise, all technical and scientific terms used herein have the same meaning as commonly understood by those of ordinary skill in the art to which this invention belongs. The terminology used in the present invention is for the purpose of describing particular embodiments only and is not intended to be limiting of the present invention.

It should also be understood that in certain methods described herein that include more than one step or action, the order of the steps or actions of the methods is not necessarily limited to the order in which the steps or actions of the methods are recited, unless the context indicates otherwise.

As used herein, the term Ras is H-Ras (e.g., having the amino acid sequence set forth as Accession: NP_001123914 in NCBI Protein Database), K-Ras (e.g., having the amino acid sequence set forth as Accession: NP_203524 in NCBI Protein Database), or N-Ras (e.g., having the amino acid sequence set forth as Accession: EAW56611 in NCBI Protein Database) and various isoforms and various active mutants thereof. Such mutants include, but are not limited to G12V, G12D, G13V, G13D and Q61R. Preferably, the mutant is G12V, G13D and Q61R of H-Ras; G12V, G12D, G13V, G13D and Q61R of K-Ras; G12V, G13V, G13D and Q61R of N-Ras. Most preferably, the mutant is G12V of K-Ras. For example, an exemplary K-Ras protein sequence can be:
1 mteyklvvvg aggvgksalt iqliqnhfvd eydptiedsy rkqvvidget clldildtag 61 qeeysamrdq ymrtgegflc vfainntksf edihhyreqi krvkdsedvp mvlvgnkcdl 121 psrtvdtkqa qdlarsygip fietsaktrq rvedafytlv reirqyrlkk iskeektpgc 181 vkikkciim

Exemplary H-Ras protein sequences can be:

1 mteyklvvvg aggvgksalt iqliqnhfvd eydptiedsy rkqvvidget clldildtag
61 qeeysamrdq ymrtgegflc vfainntksf edihqyreqi krvkdsddvp mvlvgnkcdl
121 aartvesrqa qdlarsygip yietsaktrq gvedafytlv reirqhklrk lnppdesgpg
181 cmsckcvls

Exemplary N-Ras protein sequences can be:

1 mteyklvvvg aggvgksalt iqliqnhfvd eydptiedsy rkqvvidget clldildtag
61 qeeysamrdq ymrtgegflc vfainnsksf adinlyreqi krvkdsddvp mvlvgnkcdl
121 ptrtvdtkqa helaksygip fietsaktrq gvedafytlv reirqyrmkk lnssddgtqg
181 cmglpcvvm

As used herein, the term "Nedd4-1" is an E3 ubiquitin ligase belonging to the HECT family, structurally includes an N-terminal C2 domain, an intermediate WW domain and a C-terminal HECT domain, which has, for example the amino acid sequence set forth as Accession: NP_006145 in NCBI protein database, e.g., the amino acid sequence shown below:

1 maqslrlhfa arrsntypls etsgddldsh vhmcfkrptr istsnvvqmk ltprqtalap
61 likenvqsqe rssvpssenv nkkssclqis lqptrysgyl qssnvladsd dasftcilkd
121 giyssavvdn elnavndghl vsspaicsgs lsnfstsdng syssngsdfg scasitsggs
181 ytnsvisdss sytfppsddt flggnlpsds tsnrsvpnrn ttpceifsrs tstdpfvqdd
241 lehgleimkl pvsrntkipl krysslvifp rspsttrpts ptslctllsk gsyqtshqfi
301 ispseiahne dgtsakgfls tavnglrlsk tictpgevrd irplhrkgsl qkkivlsnnt
361 prqtvcekss egyscvsvhf tqrkaatldc ettngdckpe mseiklnsds eyiklmhrts
421 aclpssqnvd cqiningele rphsqmnknh gilrrsislg gaypniscls slkhncskgg
481 psqllikfas gnegkvdnls rdsnrdctne lsnscktrdd flgqvdvply plptenprle
541 rpytfkdfvl hprshksrvk gylrlkmtyl pktsgseddn aeqaeelepg wvvldqpdaa
601 chlqqqqeps plppgweerq dilgrtyyvn hesrrtqwkr ptpqdnltda engniqlqaq
661 rafttrrqis eetesvdnre ssenweiire deatmysnqa fpsppssnl dvpthlaeel
721 narltifgns avsqpasssn hssrrgslqa ytfeeqptlp vllptssglp pgweekqder
781 grsyyvdhns rtttwtkptv qatvetsqlt ssqssagpqs qastsdsgqq vtqpseieqg
841 flpkgwevrh apngrpffid hntktttwed prlkipahlr gktsldtsnd lgplppgwee
901 rthtdgrify inhnikrtqw edprlenvai tgpavpysrd ykrkyeffrr klkkqndipn
961 kfemklrrat vledsyrrim gvkradflka rlwiefdgek gldyggvare wffliskemf
1021 npyyglfeys atdnytlqin pnsglcnedh lsyfkfigrv agmavyhgkl ldgffirpfy
1081 kmmlhkpitl hdmesvdsey ynslrwilen dpteldlrfi ideelfgqth qhelknggse
1141 ivvtnknkke yiylviqwrf vnriqkqmaa fkegffelip qdlikifden elellmcglg
1201 dvdvndwreh tkykngysan hqviqwfwka vlmmdsekri rllqfvtgts rvpmngfael
1261 ygsngpqsft veqwgtpekl prahtcfnrl dlppyesfee lwdklqmaie ntqgfdgvd

As used herein, a cancer or tumor with a Ras-positive genetic background refers to a state in which the Ras protein is mutated or continuously activated by upstream signals.

In one aspect, the present invention relates to a method of identifying a compound that regulates Ras level, the method comprising:

a. contacting a Ras-expressing cell or an in vitro ubiquitination system with a test compound;
b. selecting a compound that can increase or decrease the Ras level of the cell or system in step a.

Optionally, the method further comprising:

c. detecting whether the compound obtained in step b regulates Ras level depending on the presence of Nedd4-1 protein or not; and
d. selecting a compound that regulates the Ras level depending on the presence of the Nedd4-1 protein.

The detection method may be one conventional in the art for detecting molecular levels, including, but not limited to, microarray, ELISA, multiplex techniques, Bio-Plex®, luminex techniques, mass spectrometry, flow cytometry, Northern blotting, Southern blotting, Western blotting, PCR, and RIA.

In one aspect, the present invention relates to a method of regulating Ras level, the method comprising:

a. judging the state of Ras protein;
b. if the Ras protein is a wild-type protein or in an inactive state, regulating the Ras level by regulating the expression level of Nedd4-1;
c. if the Ras protein is a mutant protein or in an active state, regulating the Ras level by adding a compound; and
d. optionally, regulating the expression level of Nedd4-1 and adding the compound can be performed simultaneously.

The method according to the preceding, wherein the Ras level is decreased by up-regulating the level of Nedd4-1, or increased by down-regulating the level of Nedd4-1.

The method according to the preceding, wherein the level of Ras protein is decreased by adding a compound without affecting the mRNA level of Ras.

The method according to the present invention, wherein the Ras-expressing cell is a cell expressing exogenous Ras or endogenous Ras, or both. The exogenous Ras refers to Ras which is not originally present in a cell and is externally introduced into the cell through molecular biological means for expression, and the endogenous Ras refers to Ras which is originally possessed and stably inherited by the cell. In vitro Ubiquitination System is based on the in vitro ubiquitination experimental method routinely used in this field. This system does not need intact cells. The purified ubiquitin protein E1 ubiquitin activating enzyme, E2 ubiquitin binding enzyme, E3 ubiquitin ligase and the corresponding substrate protein were added to the reaction system of 50 mM Tris-hydrochloric acid (pH 7.5), 5 mM ATP, 10 mM $MgCl_2$ and 50 $\mu$M DTT for performing ubiquitination reaction. Those skilled in the art may also add or decrease specific components as desired on this basis.

The method according to the present invention, wherein in step b a compound that can decrease the Ras level of a cell in step a is selected.

The method according to the present invention, wherein the Ras is H-Ras, K-Ras or N-Ras and various mutants thereof, including but not limited to G12V, G12D, G13V, G13D and Q61R. Preferably, the mutant is G12V, G13D and Q61R of H-Ras; G12V, G12D, G13V, G13D and Q61R of K-Ras; G12V, G13V, G13D and Q61R of N-Ras. Most preferably, the mutant is G12V of K-Ras.

According to the methods of the present invention, the Ras level can be a basal level or state of a molecule in a sample, or a level or state of a molecule after a portion of the sample has been contacted with a regulator. In one embodiment, the molecule is a protein or nucleic acid molecule. In another embodiment, the molecule comprises protein, nucleic acid, lipid, sugar, carbohydrate or metabolite molecule. In yet another embodiment, the molecules are analyzed by using a method selected from the group consisting of: microarray, ELISA, multiplex techniques, Bio-Plex®, luminex techniques, mass spectrometry, flow cytometry, Northern blotting, Southern blotting, Western blotting, PCR, and RIA.

According to the method of the present invention, the interaction may be detected by immunoassay, surface plasmon resonance (SPR) (Rich and Myszka (2000) Curr. Opin. Biotechnol 11:54; Englebienne (1998) Analyst. 123:1599), isothermal titration calorimetry (ITC), or other kinetic interaction assay methods known in the art for detection (see, e.g., Paul, ed., Fundamental Immunology, 2nd ed., Raven Press, New York, pages 332-336 (1989); See also U.S. Patent No. 7, 229, 619 which describes exemplary SPR and ITC methods for calculating the binding affinity of antibodies). Instruments and methods for real-time detection and monitoring of binding rates are known and commercially available (see, BiaCore 2000, Biacore AB, Upsala, Sweden and GE Healthcare Life Sciences; Malmqvist (2000) Biochem. Soc. Trans. 27:335). It will be appreciated by those skilled in the art that the above-described methods for detecting molecular levels and methods for detecting molecular interactions may sometimes be used interchangeably, e.g., immunoblotting methods for detecting molecular levels may also be used to detect interactions between proteins, and may also be used to detect interactions of proteins with RNA, DNA molecules, or interactions of antigens and antibodies, etc.

In another aspect, the present invention relates to the compounds obtained by screening by using the method of any one of the preceding.

According to the foregoing compounds, wherein the compounds are capable of promoting ubiquitination degradation of Ras in a Nedd4-1 protein-dependent manner (i.e., the compounds can promote Ras protein degradation in the presence of the Nedd4-1 protein, but the ability of the compounds to promote Ras protein degradation is significantly diminished in the absence of the Nedd4-1 protein).

A compound according to the preceding, is selected from:

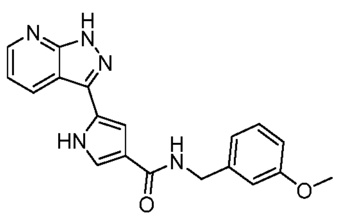 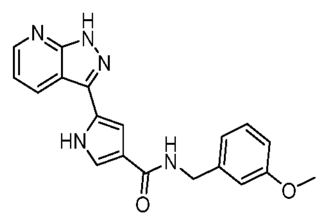 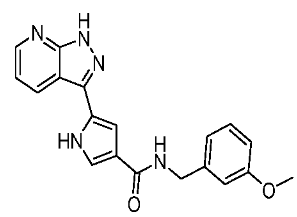

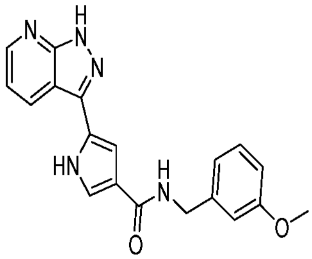 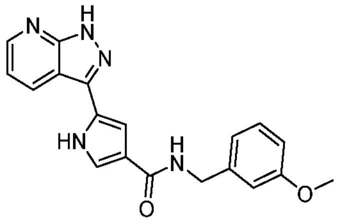 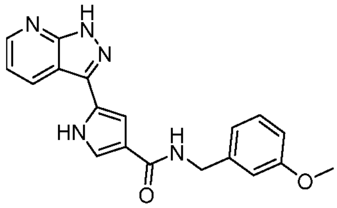

Preferably the compound is selected from:

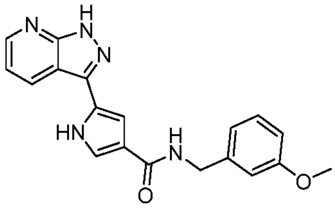,

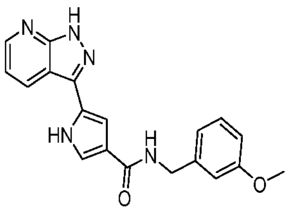,

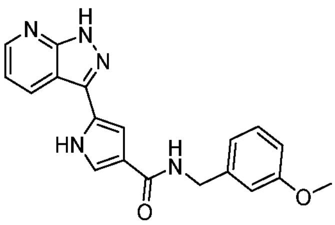.

The present invention also includes the kits employing the methods of the present invention. The kit comprises one or more reagents for detecting Ras or Nedd4-1 level, optionally, further comprises an expression plasmid expressing Ras or Nedd4-1, optionally, further comprises a host cell expressing Ras, optionally, further comprises a compound that promotes ubiquitination degradation of Ras, optionally, a compound obtained by using the method of any one of the preceding. The kit may also include suitable containers, instructions for use, and the like. Detection of Ras or Nedd4-1 level can be at the mRNA or protein level, and for detection of mRNA or protein level, other techniques and systems practiced in the art can be used, such as, for example, RT-PCR assays using primers in one or more designs; immunoassays, such as enzyme-linked immunosorbent assay (ELISA); immunoblotting, for example Western blotting or in situ hybridization and similar techniques.

As used herein, unless otherwise indicated, the term "compound" as the context applied refers to any particular chemical compound disclosed herein and comprises tautomers, positional isomers, geometric isomers, and where applicable stereoisomers thereof (including optical isomers (enantiomers) and other stereoisomers (diastereomers)), as well as pharmaceutically acceptable salts and derivatives thereof (including prodrug forms). As used herein, the term compound broadly refers to a single compound, and may include other compounds, such as stereoisomers, positional isomers, and/or optical isomers of the disclosed compounds (including racemic mixtures), as well as specific enantiomers or enantiomerically enriched mixtures. The term in this context also refers to a prodrug form of a compound that has been modified to facilitate administration and delivery of the compound to an active site.

In another aspect, the present invention provides a pharmaceutical composition comprising a compound according to any one of the preceding embodiments, a stereoisomer thereof, a prodrug thereof, or a pharmaceutically

acceptable salt or pharmaceutically acceptable solvate thereof, and a pharmaceutically acceptable carrier, diluent or excipient. For example, the active ingredients of the pharmaceutical compositions of the present invention may further comprise a compound obtained by the methods of the present invention and a molecule that regulates the expression level of Nedd4-1, the molecule regulating the expression level of Nedd4-1 includes, but not limited to, small molecule compounds, Nedd4-1 nucleic acid constructs, Nedd4-1 proteins, and the like.

Methods for preparing various pharmaceutical compositions containing a certain amount of active ingredients are known or obvious to those skilled in the art based on the disclosure of the present invention. As described in REMINGTON'S PHARMACEUTICAL SCIENCES, Martin, E.W., ed., Mack Publishing Company, 19th ed. (1995), methods of preparing the pharmaceutical compositions comprise incorporation of suitable pharmaceutical excipients, carriers, diluents, and the like.

The pharmaceutical formulations of the present invention are manufactured by known methods, including conventional mixing, dissolving or lyophilizing method. The compounds of the present invention may be formulated into pharmaceutical compositions and administered to the patients through a variety of routes suitable for the selected way of administration, for example, orally or parenterally (by intravenous, intramuscular, topical or subcutaneous routes).

As used herein, unless otherwise indicated, the term "prodrug" refers to a derivative that can hydrolyze, oxidize, or otherwise react under biological conditions (in vitro or in vivo) to provide a compound of the present invention. Prodrugs undergo this reaction only under biological conditions to become active compounds, or they are active in their non-reactive form. Prodrugs can generally be prepared by using known methods, such as those method described in 1 Burger's Medicinal Chemistry and Drug Discovery (1995) 172-178, 949-982 (Manfred E. Wolff ed., 5th ed.).

As used herein, examples of the term "pharmaceutically acceptable salts" are organic acid addition salts formed from organic acids that form pharmaceutically acceptable anions, including but not limited to formates, acetates, propionates, benzoates, maleates, fumarates, succinates, tartrates, citrates, ascorbates, $\alpha$-ketoglutarates, $\alpha$-glycerophosphate, alkylsulfonate or arylsulfonate; preferably, the alkylsulfonate is methylsulfonate or ethylsulfonate; the arylsulfonate salt is a benzenesulfonate salt or a p-toluenesulfonate salt. Suitable inorganic salts may also be formed, including, but not limited to, hydrochloride, hydrobromide, hydroiodide, nitrate, bicarbonate and carbonate, sulfate or phosphate, and the like.

Pharmaceutically acceptable salts can be obtained by using standard procedures well known in the art, for example, by reacting a sufficient amount of the basic compound with a suitable acid which provides a pharmaceutically acceptable anion.

As used herein, the term "pharmaceutically acceptable carrier" is intended to include any and all solvents, dispersion media, coatings, antibacterial agents and antifungal agents, isotonic agents and absorption delaying agents, and the like, compatible with pharmaceutical administration. Suitable carriers are described in the latest edition of Remington's Pharmaceutical Sciences, which is a standard reference in the art and is incorporated herein by reference. Preferred examples of such carriers or diluents include, but are not limited to, water, saline, Ringer's solution, dextrose solution, and 5% human serum albumin. Liposomes and non-aqueous carriers, such as immobilized oils, may also be used. The use of such media and agents for pharmaceutically active substances is well known in the art. Except insofar as any conventional media or agent is incompatible with the antibody, its use in the compositions is contemplated.

As used herein, the term "treatment" generally refers to obtaining a desired pharmacological and/or physiological effect. The effect may be prophylactic based on the complete or partial prevention of the disease or its symptoms; and/or may be therapeutic based on partial or complete stabilization or cure of the disease and/or side effects due to the disease. As used herein, "treatment" encompasses any treatment of a disease in a patient, comprising: (a) preventing a disease or symptom occurring in a patient susceptible to the disease or symptom but not yet diagnosed with the disease; (b) inhibiting the symptoms of the disease, i.e. arresting its development; or (c) relieving the symptoms of the disease, i.e., causing regression of the disease or symptoms.

In another aspect, the present invention provides a method of treating or ameliorating a disease, disorder or symptom thereof in a subject or patient (e.g., an animal, such as a human) comprising administering to a subject in need a composition comprising an effective dose (e.g., a therapeutically effective dose) of a compound or salt form thereof as described herein; and pharmaceutically acceptable carriers, wherein the composition can effectively treat or improve the disease or disorders of the subject or symptoms thereof.

As used herein, the term "nucleic acid construct" is defined herein as a single-or doublestranded nucleic acid molecule, preferably an artificially constructed nucleic acid molecule. Optionally, the nucleic acid construct further comprises one or more control sequences operably linked.

As used herein, the term "operably linked" refers to a functional spatial arrangement of two or more nucleotide regions or nucleic acid sequences. The "operably linked" may be achieved by means of genetic recombination.

As used herein, the term "vector" refers to a nucleic acid delivery vehicle into which a polynucleotide that inhibits a protein can be inserted. For example, vectors include: plasmids; phagemids; cosmids; artificial chromosomes such as yeast artificial chromosomes (YAC), bacterial artificial chromosomes (BAC), or PI-derived artificial chromosomes (PAC); phages such as $\lambda$ phage or M13 phage and animal viruses. Animal viral species used as vectors are retroviruses

(including lentiviruses), adenoviruses, adeno-associated viruses, herpes viruses (e.g., herpes simplex virus, cytomegalovirus (CMV)), poxviruses, baculoviruses, papilloma viruses, papovavirus (e.g., SV40). A vector may contain a variety of elements that control expression.

As used herein, the term "host cell" refers to a cell into which a vector is introduced, including many cell types such as prokaryotic cells such as E. coli or B. subtilis, fungal cells such as yeast cells or aspergillus, insect cells such as S2 drosophila cells or Sf9, or animal cells such as HeLa cells, SW620 cells, SW480 cells, HT-29 cells, HEK 293T cells.

As used herein, the term "effective dose" refers to a dose that can be achieved in subjects to treat, prevent, alleviate and / or relieve the disease or disorder described in the present invention.

As used herein, the term "disease and/or disorder" refers to a physical state of the subject associated with the disease and/or disorder described herein.

As used herein, the term "subject" may refer to a patient or other animal, particularly a mammal, e.g., a human, dog, monkey, cow, horse, etc., receiving a pharmaceutical composition of the present invention to treat, prevent, alleviate, and/or relieve a disease or disorder described herein.

## EXAMPLES

Hereinafter, embodiments of the present invention will be described in detail with reference to examples. Those skilled in the art will appreciate that the following examples are merely illustrative of the present invention and are not to be construed as limiting the scope of the present invention. Specific techniques or conditions not specified in the examples were performed according to techniques or conditions described in the literature in the art (see, e.g., J. Sambrook et al., Molecular Cloning A Laboratory Manual, 3rd ed., Scientific Press, translated by Huang Peitang et al.) or according to the manufacturer's instructions. The reagents or instruments used herein are conventional products that can be purchased from the market if the manufacturer is not specified.

### Example 1: Nedd4-1 degrades the Ras protein by ubiquitination modification.

#### 1. Experimental Materials and Primary Reagents

Cell lines:

Human cervical cancer cell HeLa (cat. CCL-2), purchased from ATCC. Human embryonic kidney cells HEK293T (cat. CRL-3216), purchased from ATCC. Human colon cancer cells HT-29 (cat. HTB-38), purchased from ATCC.

Vector pCMV6 (cat. PS 100001), purchased from origene.

Transfection reagents: PEI (cat. 11668-027), purchased from Invitrogen.

Dulbecco's modified medium (DMEM, Gibco, cat. 11965), purchased from ThermoFisher. Primary antibody for Western blotting:

Anti-c-Myc antibody, purchased from Santa Cruz; Anti-rat-HA antibody, purchased from Roche; Anti-Ub antibody, purchased from Santa Cruz; Anti-Flag antibody, purchased from Sigma; Anti-K-Ras antibody, purchased from Abcam; Anti-H-Ras antibody, purchased from Abcam; Anti-N-Ras antibody, purchased from Abcam; Anti-Ras antibody, purchased from Abcam; Anti-Nedd4-1 antibody, purchased from Millipore; Anti-β-actin antibody, purchased from Santa Cruz.

Secondary antibody for Western blotting:

Peroxidase conjugated goat anti-mouse IgG (H+L) and peroxidase conjugated goat anti-rabbit IgG (H+L), both purchased from Thermo Fisher.

#### 2. Experimental method

#### 2.1 Protein GST-pull down assay in vitro

The GST, GST-Nedd4-1 WT and GST-Nedd4-1 CA fusion proteins were expressed and purified by glutathione S-transferase (GST) gene fusion vector in vitro. At the same time, the GST-Flag-Ras protein was purified in vitro. According to the protein GST pull-down reaction system in vitro, the corresponding amount of purified protein was added to the TNTE 0.5% reaction buffer. The rotating shaker was incubated at 4°C for 1-2 h, then centrifuged at 2400 rpm for 1 min at 4°C, and the supernatant was discarded. TNTE 0.5% reaction buffer was added, uniformly mixing, centrifuged at 2400 rpm for 1 min at 4°C, the supernatant was discarded, and the above steps were repeated for 4 to 6 times. The reaction was stopped by adding 20 μL of 4 × loading buffer and boiled for 5 min. Samples were subjected to SDS-PAGE and Western blot analysis.

### 2.2 In vitro ubiquitination assay

In vitro ubiquitination experiments were performed according to conventional experimental methods in the art. Specifically, the fusion protein of GST-UBE1, His-UbcH7, His-Ub, GST-Nedd4-1 WT, GST- Nedd4-1 CA and GST-Flag-Ras was expressed and purified in vitro, and the corresponding amount of purified protein was added according to the protein ubiquitination reaction system in vitro. In the process of reaction, E3 was mixed with substrate and incubated on ice for 30 to 45 min, then water was added, E2, Ub, ATP and 5 × buffer were added, and E1 was added at the same time to start ubiquitination reaction, and incubated at 25°C for 50 to 60 min. After the reaction was complete, a final concentration of 1% SDS was added and boiled in boiling water for 5 min. In each group, TNTE 0.5% was added to enlarge the volume, Flag antibody was added, and protein A/G was incubated overnight on a rotating shaker at 4°C, centrifuged at 2400 rpm for 1 min at 4°C, and the supernatant was discarded. TNTE 0.5% reaction buffer was added, uniformly mixing, centrifuged at 2400 rpm for 1 min at 4°C, the supernatant was discarded, and the above steps were repeated for 3 to 4 times, adding 20 μL of 4 × loading buffer and boiled for 5 min. Samples were subjected to SDS-PAGE and Western blot analysis.

### 2.3 Preparation of cell expressed protein samples

1. After the cell culture plate was removed from the 37°C carbon dioxide incubator, the supernatant was absorbed and washed with warm PBS for one time.
2. An appropriate amount of ice-precooled lysate (PMSF in a ratio of 1:100) was added and put it in a decolorizing shaker to shake and lyse the cells for 20 min.
3. All lysates were collected into centrifuge tubes and centrifuged at 12000 rpm for 10 min at 4°C.
4. The supernatant was transferred into a new tube, 4 × loading buffer solution was added, and uniformly mixing.
5. The samples were heated in a water bath at 100°C for 5 min and placed in a refrigerator at -20°C for Western blotting analysis.

### 2.4 Co-immunoprecipitation experiment

1. 200 μL protein A/G agarose beads (mixed at 1:1) were taken, washed with TNTE 0.5% solution for 3 times, centrifuged at 3000 rpm for 30 s each time, 1% BSA was added to seal the beads for 2 h, then washed with TNTE 0.5% solution for 3 times, store at 4°C for later use.
2. 500 μL cell lysate was mixed with 0.5 μL antibody or IgG and rotationally incubated at 4°C for 2 h.
3. 20 μL protein A/G agarose beads were added and incubated at 4°C for 12 h to immunoprecipitate the labeled protein from the lysate.
4. The agarose beads/antibodies/antigen complexes were centrifuged to the bottom of the tube after 3000 rpm centrifugation at 4°C for 1 min.
5. The supernatant was discarded and the agarose beads were washed with 1 mL TNTE 0.5% solution and centrifuged at 3000 rpm for 1 min at 4°C, and the supernatant was discarded and repeated 3 times.
6. The agarose beads/antibodies/antigen complexes were resuspended in 20 μL SDS loading buffer and heated in a 100°C water bath for 5 min, the resulting samples were used for Western blot analysis.

## 3. Experimental results

The experimental results are shown in Fig. 1. Fig. 1(a) shows the detection of the interaction of endogenous Nedd4-1 with Ras proteins in HeLa cells. Fig. 1(b) shows the direct interaction of the Nedd4-1 protein with Ras protein in vitro. Fig. 1(c) shows that overexpression of Nedd4-1 protein can decrease the level of endogenous Ras protein in HeLa cells. The statistical result is the Mean ± SD of three independent experiments. Fig. 1 (d) shows that overexpression of Nedd4-1 protein can decrease exogenous Ras protein level. The corresponding plasmid combinations were transiently transfected into HEK293T cells: Flag-tagged K-Ras (F/K-Ras), H-Ras (F/H-Ras) or N-Ras (F/N-Ras) and Flag-tagged Nedd4-1 (F/Nedd4-1) or Nedd4-2 (F/Nedd4-2), wild-type (WT) or mutants C867A or C942A without catalytic activity. The whole cell lysate was used for Western blotting to detect the protein level, and the GAPDH protein level was used as the loading sample control. The statistical result was the mean ± SD of five independent experiments. Fig. 1(e) shows that Nedd4-1-mediated degradation of Ras proteins through the lysosomal pathway. Plasmid F/Nedd4-1 (WT or C867A) and different Ras were transiently transfected into HEK293T cells, and Ras protein levels was detected under conditions

of treating with or without of 20 μM proteasome inhibitor MG-132 or 120 μM lysosomal inhibitor chloroquine for 12 h. The statistical result was the Mean ± SD of three independent experiments. Fig. 1(f) shows that Nedd4-1 mediates in vivo ubiquitination of H-Ras and N-Ras. F/H-Ras or F/N-Ras, Myc/Nedd4-1 (WT or C867A) and HA/Ub were transiently transfected into HEK293T cells for ubiquitination assay in vivo. Fig. 1(g) shows that H-Ras and N-Ras were directly modified by ubiquitination with Nedd4-1 under in vitro conditions. GST/Nedd4-1 (WT or C867A) and F/H-Ras or F/N-Ras proteins were purified in vitro for performing ubiquitination reaction and ubiquitin-bound H-Ras or N-Ras was detected by Ub antibody. Fig. 1(h) shows that Nedd4-1 regulates the half-life of H-Ras proteins. As shown in the figure, F/H-Ras, F/Nedd4-1, control shRNA, and Nedd4-1 shRNA plasmids were transiently transfected into HEK293T cells. The cells were collected after cycloheximide (CHX) treating at different time points to detect the level of F/H-Ras protein in the whole cell lysate, and Image Lab software (Bio-Rad) was used to quantify the protein with GAPDH as the internal reference. F/H-Ras at each time point versus the amount of protein at zero was displayed by a line chart. Fig. 1(i) shows that Nedd4-1 regulates the half-life of N-Ras proteins. As shown in the figure, F/N-Ras, F/Nedd4-1, control shRNA, and Nedd4-1 shRNA plasmids were transiently transfected into HEK293T cells. The cells were collected after cycloheximide (CHX) treating at different time points to detect the level of F/N-Ras protein in the whole cell lysate, and Image Lab software (Bio-Rad) was used to quantify the protein with GAPDH as the internal reference. F/N-Ras at each time point versus the amount of protein at zero was displayed by a line chart. Fig. 1(j) shows that overexpression of Nedd4-1 in HT-29 cells can decrease endogenous WT K-Ras protein level, and knocking down Nedd4-1 can upregulate endogenous WT K-Ras protein level. Fig. 1(k) shows that K-Ras G12V mutant did not significantly degrade when F/Nedd4-1 was overexpressed. As shown in the figure, F/K-Ras (WT or G12V) and F/Nedd4-1 (WT or C867A) were transiently transfected into HEK293T cells, and F/K-Ras protein levels were measured. Fig. 1(l) shows that K-Ras G12V mutation attenuates Nedd4-1-mediated K-Ras ubiquitination in vivo. F/K-Ras (WT or V12), Myc/Nedd4-1 (WT or C867A) and HA/Ub were transiently transfected into HEK293T cells for ubiquitination assay in vivo. Fig. 1(m) shows that K-Ras G12V mutation attenuates Nedd4-1-mediated K-Ras ubiquitination in vitro. GST/Nedd4-1 (WT or C867A) and F/K-Ras (WT or V12) proteins were purified in vitro for performing ubiquitination reaction and ubiquitin-bound K-Ras was detected by Ub antibody.

## Example 2: Dot blotting high-throughput screening of compounds promoting K-Ras-G12V protein degradation, further detected through western blotting and finally obtaining target compounds depending on Nedd4-1 for K-Ras-G12V degradation.

### 1. Experimental Materials and Primary Reagents

Cell lines:

Human colon cancer cell SW620 (cat. CCL-227), purchased from ATCC. Human embryonic kidney cells HEK293T (cat. CRL-3216), purchased from ATCC. Vector pCMV6 (cat. PS 100001), purchased from origene.

Transfection reagents: PEI (cat. 11668-027), purchased from Invitrogen.

Dulbecco's modified medium (DMEM, Gibco, cat. 11965), purchased from Thermo Fisher. RPMI medium 1640 (Gibco, cat. 31800022), purchased from Thermo Fisher.

Primary antibody for Western:

Anti-Flag antibody, purchased from Sigma; Anti-K-Ras antibody, purchased from Abcam; Anti-Nedd4-1 antibody, purchased from Millipore; Anti-β-actin antibody, purchased from Santa Cruz.

Secondary antibody for Western blotting:

Peroxidase conjugated goat anti-mouse IgG (H+L) and peroxidase conjugated goat anti-rabbit IgG (H+L), both purchased from Thermo Fisher.

### 2. Experimental method

Dot blot high throughput screening of compounds promoting K-Ras-G12V protein degradation

1) Cell transfection: HEK293T cells with a density of 70% - 90% were prepared, the plasmid to be transfected (the plasmid expressing N-terminal Flag (amino acid sequence DYKDDDDK)-tagged K-Ras-G12V was constructed by inserting K-Ras-G12V gene into commercially purchased pCMV6 expression vector (for example, pCMV6 general vector purchased from origene) through ordinary PCR technique cloning, and the plasmid expressing N-terminal

Flag-tagged Nedd4-1 was constructed by inserting Nedd4-1 gene into commercially purchased pCMV6 expression vector through ordinary PCR technique cloning) was diluted to 0.1 μg/μL, and the transfection buffer was prepared according to the transfection system, followed by shaking in a vortex mixer for 10 s, the cells to be transfected were stood at room temperature for 30 min, during which the cells to be transfected were put in DMEM medium without FBS, which was added drop by drop after 30 min, and the medium was changed after 6-10 h.

2) Cell plating: the transfected HEK293T cells were digested and suspended and seeded into 96-well plates and cultured in a 37°C, 5% CO2 incubator at a cell density of about 70% when seeded the next day.

3) Cell administration: after 24 hours of culture, various drug stock solutions were diluted with a DMEM culture medium containing 10% FBS to obtain a drug solution with a final concentration of 20 μM; the stock solution from the 96-well plate was discarded, then the prepared drug solution was added, with taking the DMSO group as a blank control; after the drug was added, it was transferred to the CO2 incubator at 37°C to continue the culture.

4) Detection of K-Ras protein level: 24 h after administration, liquid in the well was discarded, the cells were routinely collected, and the cell lysate was added to extract the total cell protein. The protein concentration was determined by BCA method. The 30 μg protein sample was placed on the nitrocellulose membrane. After the sample was completely adsorbed on the membrane, the membrane was sealed in turn, and the membrane was incubated overnight with Flag antibody (1:2000) and internal reference antibody β-actin (1:2000) at 4°C, respectively. After TBST rinsing 5 min × 3 times, the corresponding horseradish peroxidase labelled secondary antibody (1:5000) was added to incubate for 1-2 h, TBST washing membrane 5 min × 3 times, and the chemiluminescence images were collected by Image Lab software.

5) Calculation of single point K-Ras protein degradation rate: quantitative and calibration analysis on Flag-K-Ras and β-actin were performed by Image-Lab software, and then the intracellular Flag-K-Ras degradation rate was calculated according to the following formula:

$$\text{single point Flag-K-Ras protein degradation rate (\%)} = \text{(control group-administration group)/control group} \times 100\%.$$

Compounds that can decrease the level of exogenous K-Ras-G12V were screened in HEK293T cells

1) Cell plating: HEK293T cells in logarithmic growth phase were inoculated into 12-well plates and cultured in 37°C and 5% CO2 incubator. The best inoculation amount was at a cell density of 70% - 90% when transfected the next day.

2) Cell transfection and administration: after 24 hours of culture, the plasmid to be transfected (the plasmid expressing N-terminal Flag (amino acid sequence DYKDDDDK)-tagged K-Ras-G12V was constructed by inserting K-Ras-G12V gene into commercially purchased pCMV6 expression vector through ordinary PCR technique cloning, and the plasmid expressing N-terminal Flag-tagged Nedd4-1 was constructed by inserting Nedd4-1 gene into commercially purchased pCMV6 expression vector through ordinary PCR technique cloning) was diluted to 0.1 μg/μL, and the transfection buffer was prepared according to the transfection system, followed by shaking in a vortex mixer for 10 s, the cells to be transfected were stood at room temperature for 30 min, during which the cells to be transfected were put in DMEM medium without FBS, which was added drop by drop after 30 min, and the medium was changed after 6-10 h. Various drug stock solutions were diluted with a DMEM culture medium containing 10% FBS to obtain a drug solution with a final concentration of 20 μM; the stock solution from the 12-well plate was discarded, then the prepared drug solution was added, with taking the DMSO group as a blank control; after the drug was added, it was transferred to the CO2 incubator at 37°C to continue the culture.

3) Detection of K-Ras protein level: 24 hours after administration, liquid in the well was discarded, the cells were routinely collected, the cells were rinsed twice with 1 × PBS, and the cell lysate was added to extract the total cell protein. The protein concentration was determined by BCA method. 30 μg protein samples were taken for SDS-polyacrylamide gel electrophoresis, the membrane was sealed in turn, and the membrane was incubated overnight with Flag antibody (1:2000) and internal reference antibody β-actin (1:2000) at 4°C, respectively. After TBST rinsing 5 min × 3 times, the corresponding horseradish peroxidase labelled secondary antibody (1:5000) was added to incubate for 1-2 h, TBST washing membrane 5 min × 3 times, and the chemiluminescence images were collected by Image Lab software.

4) Calculation of single point K-Ras protein degradation rate: quantitative and calibration analysis on Flag-K-Ras and β-actin were performed by Image-Lab software, and then the intracellular Flag-K-Ras degradation rate was calculated according to the following formula:

$$\text{single point Flag-K-Ras protein degradation rate (\%)} = \text{(control group-administration group)/control group} \times 100\%.$$

Compounds that can decrease the level of endogenous K-Ras-G12V were screened in SW620 cells

1) Cell plating: SW620 cells in logarithmic growth phase were inoculated into 24-well plates with $2 \times 10^4$/well and cultured in 37°C and 5% CO2 incubator.
2) Cell administration: after 24 hours of culture, various drug stock solutions were diluted with a RPMI-1640 culture medium containing 10% FBS to obtain a drug solution with a final concentration of 20 μM; the stock solution from the 24-well plate was discarded, then the prepared drug solution was added, with taking the DMSO group as a blank control; after the drug was added, it was transferred to the CO2 incubator at 37°C to continue the culture.
3) Detection of K-Ras protein level: 24 hours after administration, liquid in the well was discarded, the cells were routinely collected, the cells were rinsed twice with 1 × PBS, and the cell lysate was added to extract the total cell protein. The protein concentration was determined by BCA method. 30 μg protein samples were taken for SDS-polyacrylamide gel electrophoresis, the membrane was sealed in turn, and the membrane was incubated overnight with K-Ras antibody (1:1000) and internal reference antibody β-actin (1:2000) at 4°C, respectively. After TBST rinsing 5 min × 3 times, the corresponding horseradish peroxidase labelled secondary antibody (1:5000) was added to incubate for 1-2 h, TBST washing membrane 5 min × 3 times, and the chemiluminescence images were collected by Image Lab software.
4) Calculation of single point K-Ras protein degradation rate: quantitative and calibration analysis on K-Ras and β-actin were performed by Image-Lab software, and then the intracellular K-Ras degradation rate was calculated according to the following formula:

single point K-Ras protein degradation rate (%) = (control group-administration

### 3. Experimental results

The results are shown in Fig. 2. Fig. 2(a) shows that 28 compounds were screened by dot-blotting high-throughput screening, which overexpressed Flag-K-Ras-G12V and Flag-Nedd4-1-WT in 293T cells, and 7 compounds were screened by dosing treatment to decrease the level of exogenous K-Ras-G12V protein. Fig. 2(b) shows that 7 compounds (structures shown in the table below) that can decrease exogenous K-Ras-G12V were further screened in SW620 cells and 3 compounds that can decrease endogenous K-Ras-G12V protein levels were selected. Fig. 2(c) shows that 3 compounds after endogenous and exogenous screening were tested to determine whether the decrease of K-Ras-G12V protein level by the 3 compounds is dependent on the presence of Nedd4-1 protein. Finally, the compound No. 3 (compound code: ZT-10-158-01) was selected for further experiments.

| Compound No. | Compound Code | Compound Structure |
|---|---|---|
| 1 | ZT-10-151-01 | 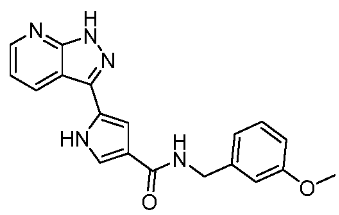 |
| 2 | ZT-10-154-01 | 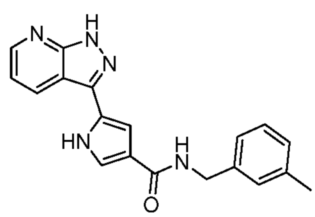 |

(continued)

| Compound No. | Compound Code | Compound Structure |
|---|---|---|
| 3 | ZT-10-158-01 | 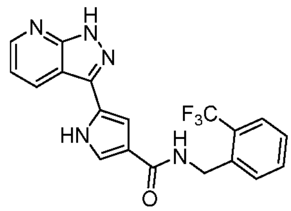 |
| 4 | ZT-11-018-01 | 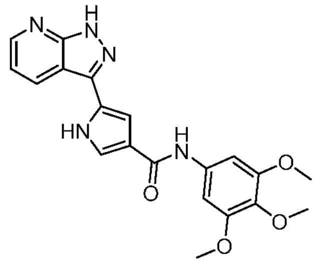 |
| 5 | ZT-11-021-01 | 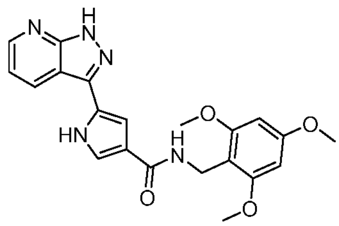 |
| 6 | ZT-11-025-01 | 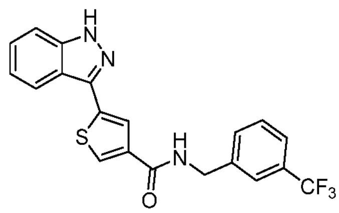 |
| 7 | ZT-11-030-01 | 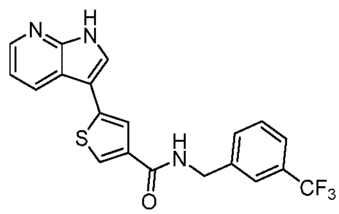 |

## Example 3: Small molecule compound ZT-10-158-01 inhibited K-Ras-G12V protein levels in a positive concentration-dependent correlation

### 1. Experimental Materials and Primary Reagents

Cell lines:

Human colon cancer cell SW620 (cat. CCL-227), purchased from ATCC. Human embryonic kidney cells HEK293T (cat. CRL-3216), purchased from ATCC.

Vector pCMV6 (cat. PS 100001), purchased from origene.

Transfection reagents: PEI (cat. 11668-027), purchased from Invitrogen.

Dulbecco's modified medium (DMEM, Gibco, cat. 11965), purchased from ThermoFisher. RPMI 1640 medium (Gibco, cat. 31800022), purchased from ThermoFisher.

Primary antibody for Western blotting:

Anti-Flag antibody, purchased from Sigma; Anti-K-Ras antibody, purchased from Abcam; Anti-Nedd4-1 antibody, purchased from Millipore; Anti-β-actin antibody, purchased from Santa Cruz.

Secondary antibody for Western blotting:

Peroxidase conjugated goat anti-mouse IgG (H+L) and peroxidase conjugated goat anti-rabbit IgG (H+L), both purchased from Thermo Fisher.

**2. Experimental method**

Inhibition of endogenous K-Ras-G12V levels in SW620 cells

1) Cell plating: SW620 cells in logarithmic growth phase were inoculated into 24-well plates with $2 \times 10^4$/well and cultured in 37°C and 5% CO2 incubator.
2) Cell administration: after 24 hours of culture, various drug stock solutions were diluted with a RPMI-1640 culture medium containing 10% FBS to obtain a drug solution with a final concentration of 2.5 μM, 5 μM, 10 μM, 15 μM, 20 μM, respectively; the stock solution from the 24-well plate was discarded, then the prepared drug solution was added, with taking the DMSO group as a blank control; after the drug was added, it was transferred to the $CO_2$ incubator at 37°C to continue the culture.
3) Detection of K-Ras protein level: 24 hours after administration, liquid in the well was discarded, the cells were routinely collected, the cells were rinsed twice with 1 × PBS, and the cell lysate was added to extract the total cell protein. The protein concentration was determined by BCA method. 30 μg protein samples were taken for SDS-polyacrylamide gel electrophoresis, the membrane was sealed in turn, and the membrane was incubated overnight with K-Ras antibody (1:1000) and β-actin internal reference antibody (1:2000) at 4°C, respectively. After TBST rinsing 5 min × 3 times, the corresponding horseradish peroxidase labelled secondary antibody (1:5000) was added to incubate for 1-2 h, TBST washing membrane 5 min × 3 times, and the chemiluminescence images were collected by Image Lab software.
4) Calculation of single point K-Ras protein degradation rate: quantitative and calibration analysis on K-Ras and β-actin were performed by Image-Lab software, and then the intracellular K-Ras degradation rate was calculated according to the following formula:

$$\text{single point K-Ras protein degradation rate (\%)} = \text{(control group-administration group)/control group} \times 100\%.$$

Inhibition of exogenous K-Ras-G12V levels in HEK293T cells

1) Cell plating: HEK293T cells in logarithmic growth phase were inoculated into 12-well plates and cultured in 37°C and 5% CO2 incubator. The best inoculation amount was at a cell density of 70% - 90% when transfected the next day.
2) Cell transfection and administration: after 24 hours of culture, the plasmid to be transfected (the plasmids are constructed by commercial purchased plasmid vectors and conventional molecular biological methods) was diluted to 0.1 μg/μL, and the transfection buffer was prepared according to the transfection system, followed by shaking in a vortex mixer for 10 s, the cells to be transfected were stood at room temperature for 30 min, during which the cells to be transfected were put in DMEM medium without FBS, which was added drop by drop after 30 min, and the medium was changed after 6-10 h. When changing the medium, various drug stock solutions were diluted with a DMEM culture medium containing 10% FBS to obtain a drug solution with a final concentration of 2.5 μM, 5 μM, 10 μM, 15 μM, 20 μM, respectively; the stock solution from the 12-well plate was discarded, then the prepared drug solution was added, with taking the DMSO group as a blank control; after the drug was added, it was transferred to the CO2 incubator at 37°C to continue the culture.
3) Detection of K-Ras protein level: 24 hours after administration, liquid in the well was discarded, the cells were routinely collected, the cells were rinsed twice with 1 × PBS, and the cell lysate was added to extract the total cell protein. The protein concentration was determined by BCA method. 30 μg protein samples were taken for SDS-polyacrylamide gel electrophoresis, the membrane was sealed in turn, and the membrane was incubated overnight with Flag antibody (1:2000) and β-actin internal reference antibody (1:2000) at 4°C, respectively. After TBST rinsing 5 min × 3 times, the corresponding horseradish peroxidase labelled secondary antibody (1:5000) was added to incubate for 1-2 h, TBST washing membrane 5 min × 3 times, and the chemiluminescence images were collected

by Image Lab software.

4) Calculation of single point K-Ras protein degradation rate: quantitative and calibration analysis on Flag-K-Ras and β-actin were performed by Image-Lab software, and then the intracellular Flag-K-Ras degradation rate was calculated according to the following formula:

single point Flag-K-Ras protein degradation rate (%) = (control group-administration group)/control group × 100%.

### 3. Experimental results

As shown in Fig. 3, the experimental results showed that compound ZT-10-158-01 was effective in decreasing K-Ras protein levels in cells, and in a positive concentration-dependent correlation.

## Example 4: the treatment of small molecular compound ZT-10-158-01 can effectively inhibit the colony forming ability of cancer cells on soft agar.

### 1. Experimental Materials and Primary Reagents

Cell lines:

Human colon cancer cell SW620 (cat. CCL-227), purchased from ATCC.
Human colon cancer cells HT-29 (cat. HTB-38), purchased from ATCC.
Human breast cancer cells MCF-7 (cat. HTB-22), purchased from ATCC.
Agar powder (cat. A100637), purchased from Sangon.
Dulbecco's modified medium (DMEM, Gibco, cat. 11965), purchased from ThermoFisher.

### 2. Experimental method

1) Bottom agar

i. 1% agar was prepared and sterilized under high pressure. 1% Agar was heated and melted in a boiling water bath before use, then cooled in a 40°C water bath (standing for at least 30 minutes and cooling the agar to 40°C); and 2 × DMEM/FBS was placed in a warm bath at the same time;

ii. 0.5% bottom agar was prepared by mixing 1% agar and 2 × DMEM/FBS in a sterile tube at a volume ratio of 1:1. The mixture was quickly added to a 6-well plate, 1.5 mL per well, gently shake it well, and it was stood at room temperature to be solidified (after solidification, it can be stored at 4°C for 1 week. Leave at room temperature for at least 30 minutes before use to restore it to room temperature).

2) Top agar

i. A final concentration of 0.3% agar + 1 × DMEM/FBS mixture was prepared in sterile tubes by using 1% agar at a temperature of 40°C, 2 × DMEM/FBS and sterile water. The prepared mixed solution was put into a 40°C water bath kettle for later use;

ii. The cells were digested by trypsin and counted, and the cell density was adjusted according to the needs of the experiment;

3) The prepared 0.3% agar + 1 × DMEM/FBS mixed solution were taken out from a 40°C water bath kettle, the corresponding number of cells and a certain amount of DMSO/ZT-10-158-01 were added, gently blew with a gun, and were added on the solidified bottom agar with 1.5 mL per well;

4) Cell well plates were cultured in 5% $CO_2$ cell incubator at 37°C for 10-30 days, every 3-6 days, supplemented with fresh medium, 200 μL per well.

5) The cell clone formation in agar was observed every day. When the cell clone size was large enough to take pictures, the cell clone formation number and clone size between the DMSO group and the experimental group treated with ZT-10-158-01 were observed and compared.

### 3. Experimental results

As shown in Fig. 4, the experimental results show that compound ZT-10-158-01 can selectively inhibit the colony formation of tumor cells that need Ras protein to activate signal pathway, such as MCF-7 cells and SW620 cells, but has no significant inhibitory effect on the colony formation of HT-29 cells with Raf kinase mutation downstream of Ras signal pathway, which no longer need Ras protein to activate this signal pathway.

Although specific embodiments of the present invention have been described in detail, those skilled in the art will appreciate that the present invention is not limited thereto. Various modifications and alterations to those details may be made in light of all the teachings disclosed which are within the scope of the present invention. The full scope of the present invention is indicated by the appended claims and any equivalents thereof.

## Claims

**1.** A method of identifying a compound that regulates Ras level, the method comprising:

a. contacting a Ras-expressing cell or an in vitro ubiquitination system with a test compound;
b. selecting a compound that can increase or decrease the Ras level of the cell or system in step a, preferably selecting a compound that can decrease the Ras level of the cell or system in step a;

optionally, the method further comprising:

c. detecting whether the compound obtained in step b regulates the Ras level depending on the presence of Nedd4-1 protein or not; and
d. selecting a compound that regulates the Ras level depending on the presence of the Nedd4-1 protein.

**2.** The method of claim 1, wherein the Ras-expressing cell or system is a cell or system that expresses exogenous Ras or endogenous Ras, or both.

**3.** A method of regulating Ras level in a cell, the method comprising:

a. contacting a compound that regulates ubiquitination degradation of Ras with a Ras-expressing cell or an in vitro ubiquitination system, optionally the activity of the compound that regulates ubiquitination degradation of Ras is Nedd4-1 dependent or independent;
b. optionally, further regulating the level of Nedd4-1 in a cell, optionally regulating the level of Nedd4-1 comprising: a. gene editing, b. overexpression, c. RNA interference, d. a small molecule compound, and e. an antibody.

**4.** A method of regulating Ras level, the method comprising:

a. judging the state of Ras protein;
b. if the Ras protein is a wild-type protein or in an inactive state, regulating the Ras level by regulating the expression level of Nedd4-1; and
c. if the Ras protein is a mutant protein or in an active state, regulating the Ras level by adding a compound; wherein

optionally, regulating the expression level of Nedd4-1 and adding the compound can be performed simultaneously;
optionally, the Ras level is decreased by up-regulating the level of Nedd4-1, or increased by down-regulating the level of Nedd4-1;
optionally, the level of Ras protein is decreased by adding a compound without affecting the mRNA level of Ras.

**5.** The method of any one of the preceding claims, wherein the Ras is selected from H-Ras, K-Ras or N-Ras or a mutant thereof, preferably the mutant is selected from G12V, G12D, G13V, G13D and Q61R, more preferably the mutant is G12V.

**6.** A compound screened by using the method of claim 1 or 2, or an isomer, prodrug, pharmaceutically acceptable salt or solvate thereof, preferably wherein the compound is capable of promoting ubiquitination degradation of Ras in a Nedd4-1 protein dependent manner, optionally the compound is selected from:

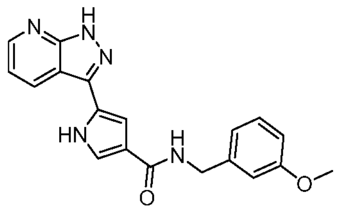

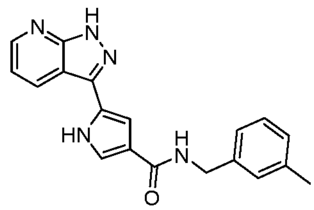

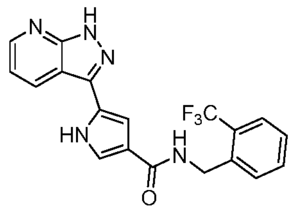

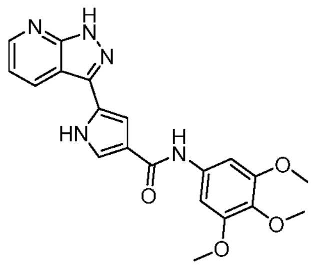

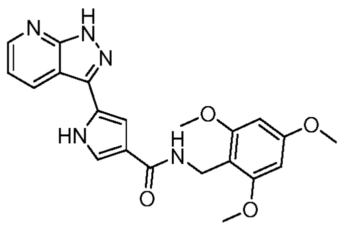

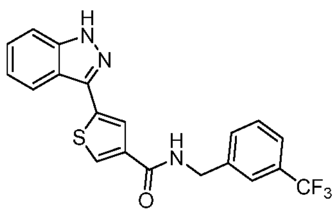

preferably the compound is selected from:

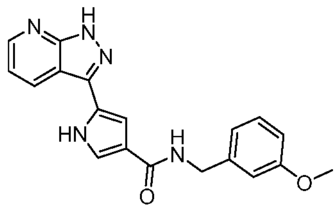

,

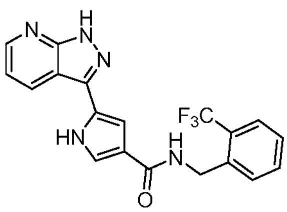

,

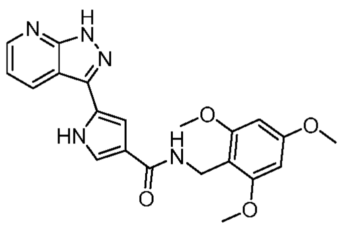

.

**7.** A method of identifying a compound for preventing or treating tumors, the method comprising:

a. contacting a tumor cell line in vitro with the compound of claim 6, and
b. selecting a compound capable of inhibiting or decreasing the colony forming ability of the cell as a candidate drug,

optionally, the tumor cell line has a Ras positive genetic background.

**8.** A kit comprising one or more reagents for detecting Ras or Nedd4-1 level, optionally, further comprising an expression plasmid expressing Ras or Nedd4-1, optionally, further comprising a host cell expressing Ras, optionally, further comprising a compound promoting ubiquitination degradation of Ras, optionally, the compound being a compound obtained by using the method of any one of the preceding claims of the invention.

**9.** Use of a compound that regulates expression level of Nedd4-1 in preparing a drug for preventing or treating tumors, optionally the compound that regulates the expression level of Nedd4-1 comprising, but not limited to, nucleic acid constructs, small molecule compounds, antibodies, and the like; preferably, the compound being the compound of claim 6.

**10.** Use of a compound that promotes ubiquitination degradation of Ras protein in preparing a drug for preventing or treating tumors, preferably, the compound that promotes ubiquitination degradation of Ras protein being Nedd4-1 dependent, or binding to Nedd4-1, optionally, the compound that regulates the expression level of Nedd4-1 comprising, but not limited to, nucleic acid constructs, small molecule compounds, antibodies, and the like; preferably, the compound being the compound of claim 6.

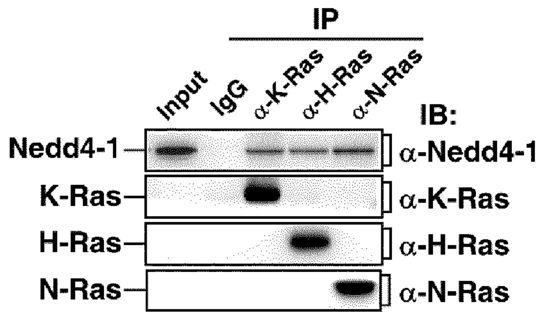


Fig. 1 (a)

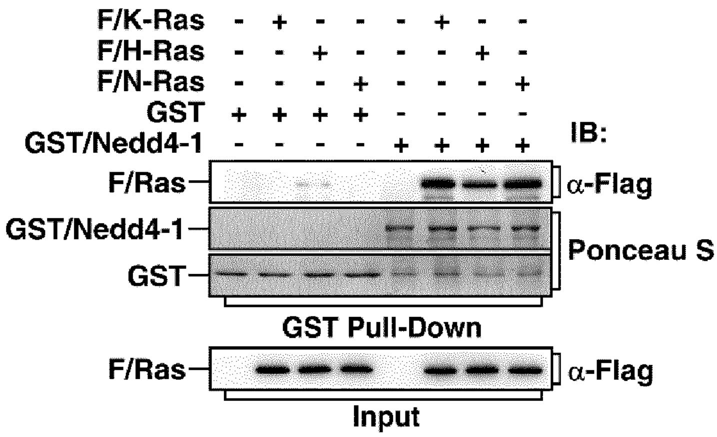


Fig. 1 (b)

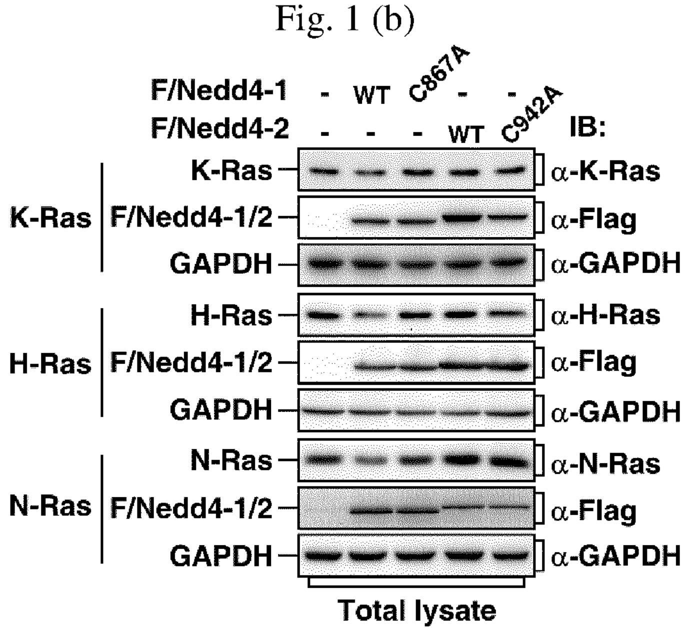


Fig. 1 (c)

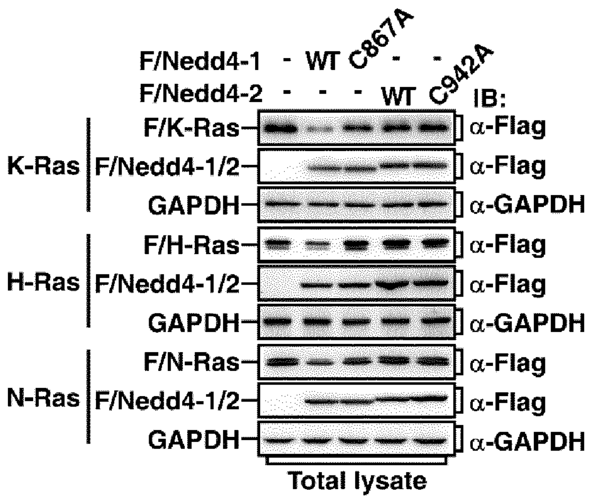


Fig. 1(d)

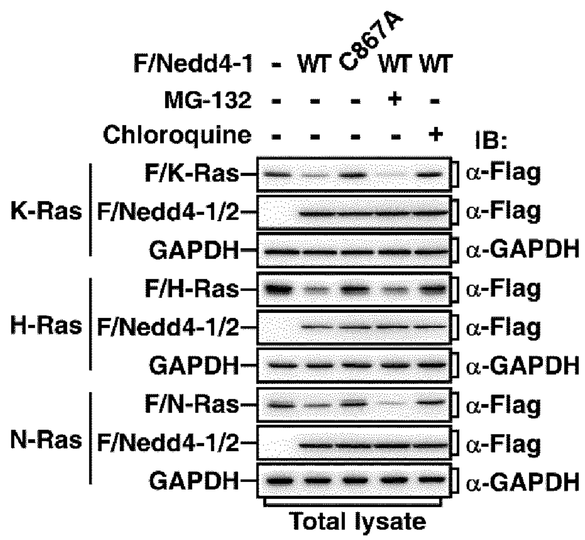


Fig. 1(e)

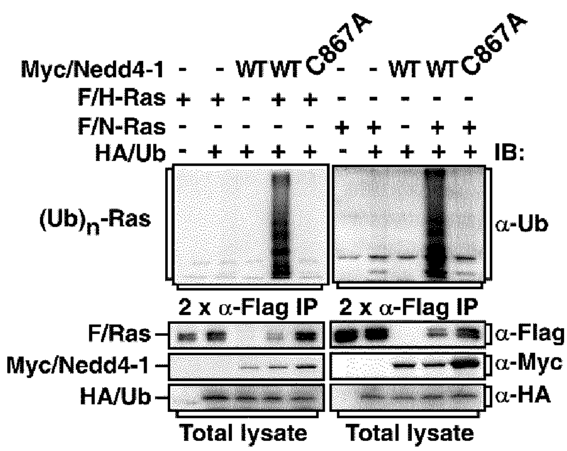


Fig. 1(f)

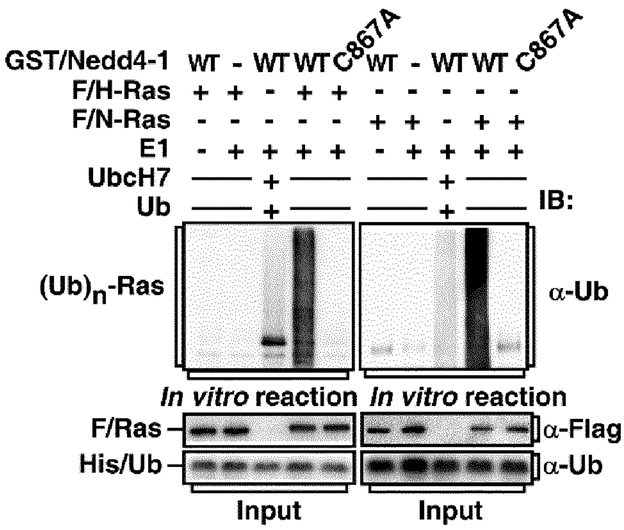


Fig. 1 (g)

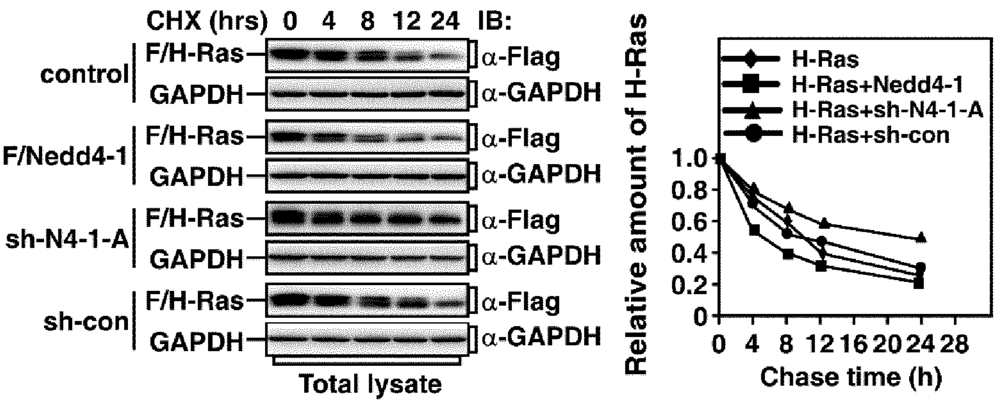


Fig. 1 (h)

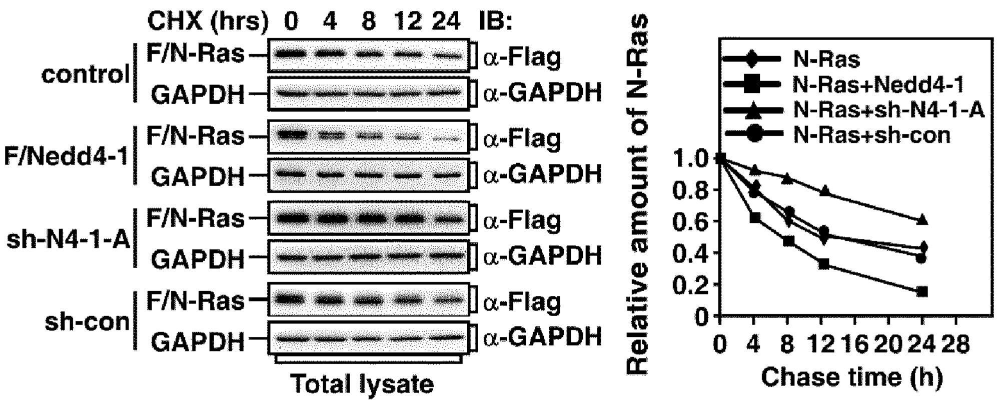


Fig. 1 (i)

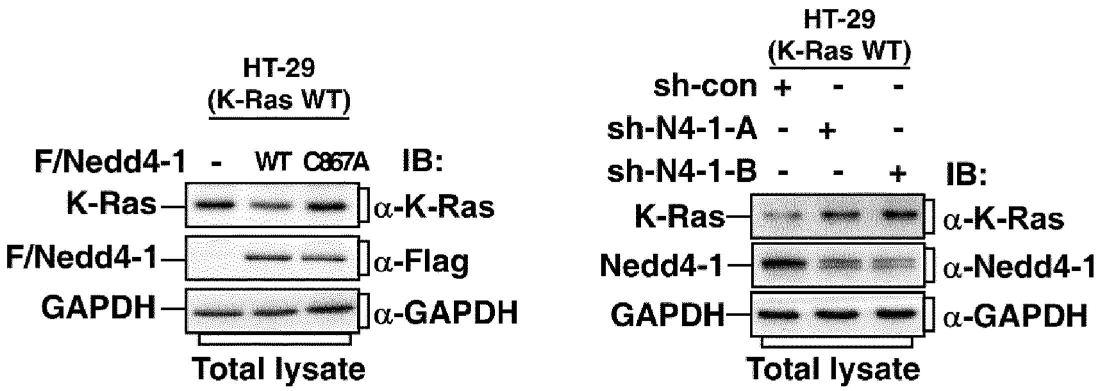


Fig. 1 (j)

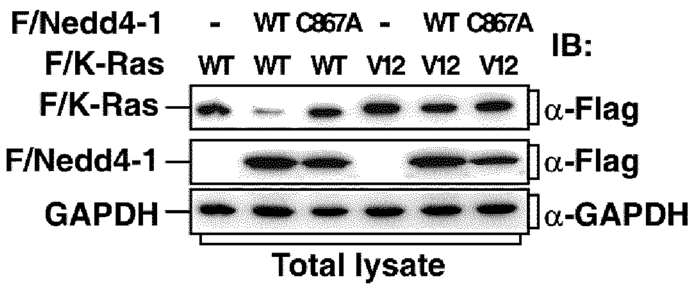


Fig. 1 (k)

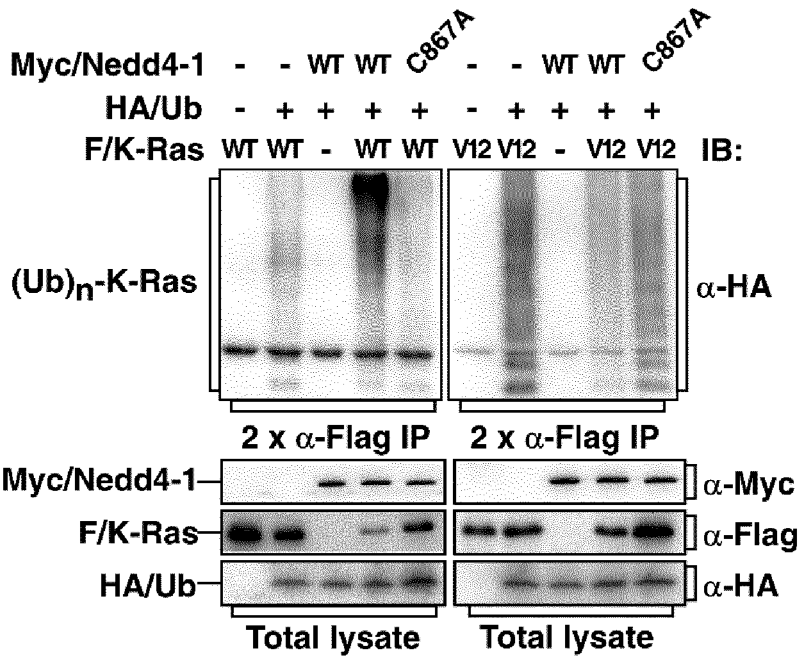


Fig. 1 (l)

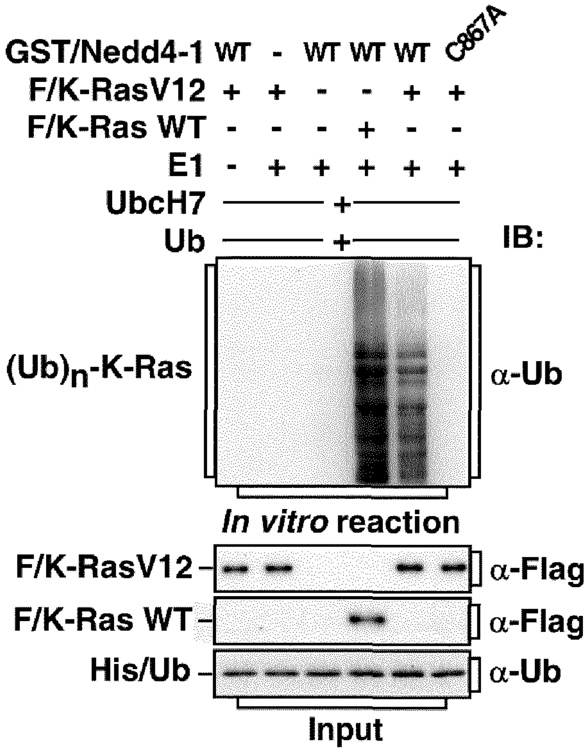
GST/Nedd4-1 WT - WT WT WT C867A
F/K-RasV12 + + - - + +
F/K-Ras WT - - - + - -
E1 - + + + + +
UbcH7 +
Ub +
IB:
(Ub)n-K-Ras
α-Ub
In vitro reaction
F/K-RasV12
α-Flag
F/K-Ras WT
α-Flag
His/Ub
α-Ub
Input

Fig. 1 (m)

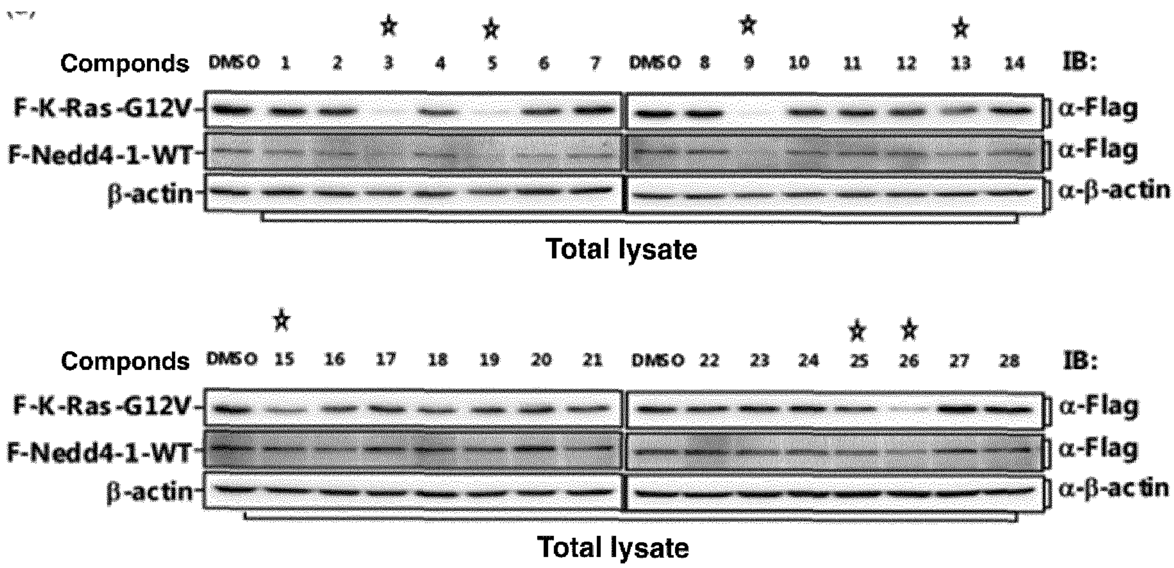
Componds DMSO 1 2 3 4 5 6 7 DMSO 8 9 10 11 12 13 14
IB:
F-K-Ras-G12V
α-Flag
F-Nedd4-1-WT
α-Flag
β-actin
α-β-actin
Total lysate
Componds DMSO 15 16 17 18 19 20 21 DMSO 22 23 24 25 26 27 28
IB:
F-K-Ras-G12V
α-Flag
F-Nedd4-1-WT
α-Flag
β-actin
α-β-actin
Total lysate

Fig. 2(a)

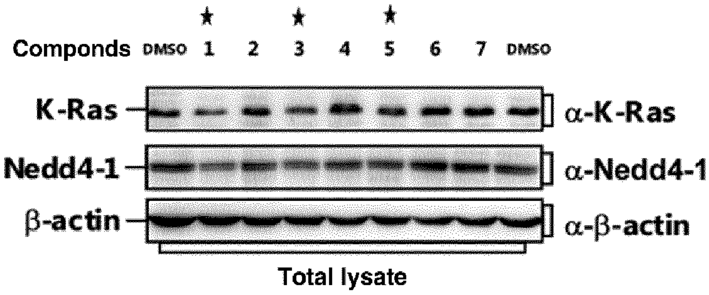


Fig. 2 (b)

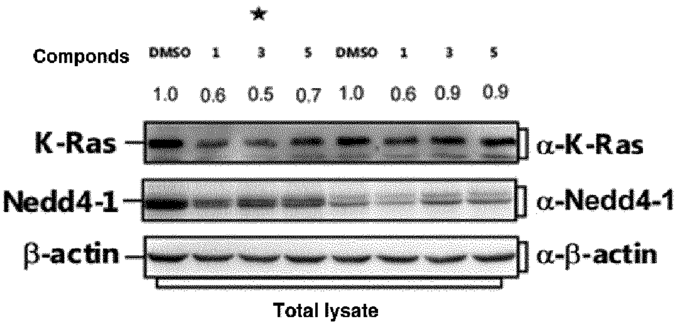


Fig. 2 (c)

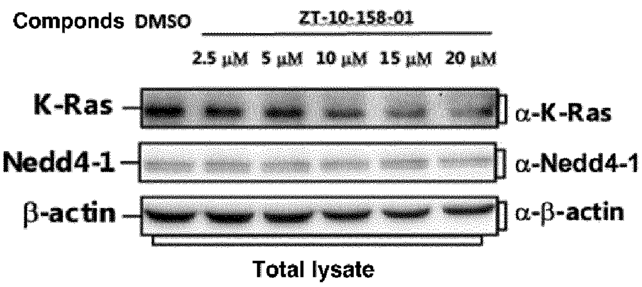


Fig. 3(a)

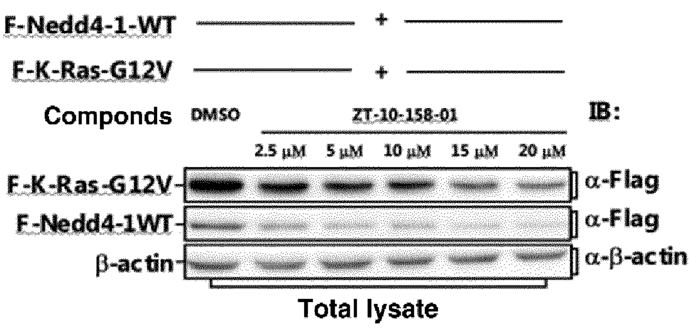


Fig. 3(b)

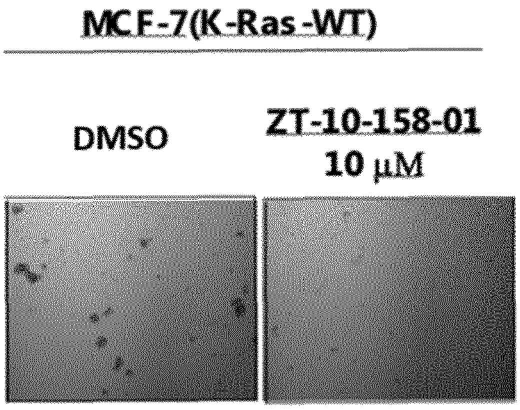


Fig. 4 (a)

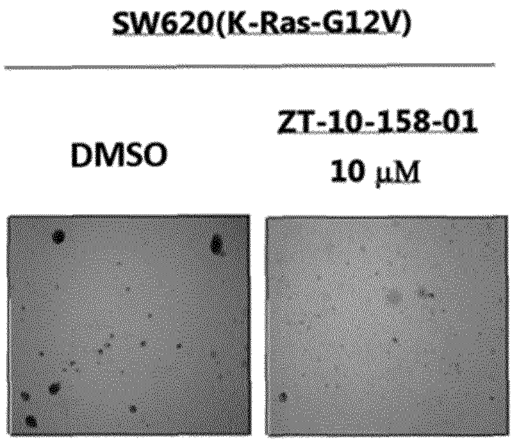


Fig. 4 (b)

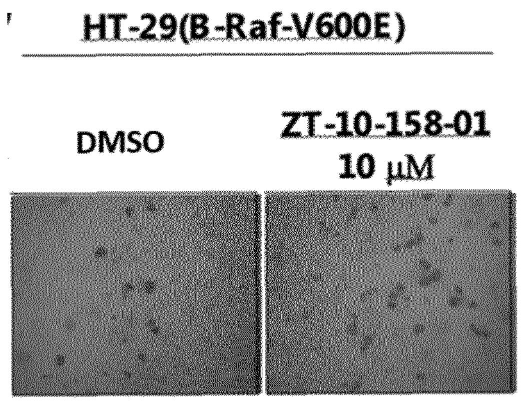


Fig. 4 (c)

**INTERNATIONAL SEARCH REPORT**

International application No. **PCT/CN2019/084176**

**A. CLASSIFICATION OF SUBJECT MATTER**

C12Q 1/02(2006.01)i; A61K 31/437(2006.01)i; A61P 35/00(2006.01)i

According to International Patent Classification (IPC) or to both national classification and IPC

**B. FIELDS SEARCHED**

Minimum documentation searched (classification system followed by classification symbols)

C12Q, A61K, A61P

Documentation searched other than minimum documentation to the extent that such documents are included in the fields searched

Electronic data base consulted during the international search (name of data base and, where practicable, search terms used)

CNMED, CNABS, CPEA, DWPI, SIPOABS, AUABS, TWMED, ILABS, TWABS, HKABS, MOABS, SGABS, CNKI, NCBI, EMBL, GoogleScholar: Nedd4-1, Ras, 肿瘤, cancer, tumor

**C. DOCUMENTS CONSIDERED TO BE RELEVANT**

| Category* | Citation of document, with indication, where appropriate, of the relevant passages | Relevant to claim No. |
|---|---|---|
| PX | CN 108546731 A (XIAMEN UNIVERSITY) 18 September 2018 (2018-09-18)<br>claims 1-10 | 1-10 |
| X | CN 107951879 A (WUHAN ZHENZHI BIOTECHNOLOGY CO., LTD.) 24 April 2018 (2018-04-24)<br>the abstract, and claims 7-9 | 1-3, 6-8 |
| Y | CN 107951879 A (WUHAN ZHENZHI BIOTECHNOLOGY CO., LTD.) 24 April 2018 (2018-04-24)<br>the abstract, and claims 7-9 | 4, 5, 9, 10 |
| Y | ZENG, Taoling et al. "Impeded Nedd4-1-Mediated Ras Degradation Underlies Ras-Driven Tumorigenesis"<br>*Cell Reports,* Vol. 7, 08 May 2014 (2014-05-08),<br>the abstract | 4, 5, 9, 10 |
| A | CN 103429757 A (MIAMI MICE RES CORPORATION CORRINNE LOBE) 04 December 2013 (2013-12-04)<br>the abstract, and claims 1-7 | 1-10 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 June 2019** | **29 July 2019** |

| Name and mailing address of the ISA/CN | Authorized officer |
|---|---|
| **National Intellectual Property Administration, PRC (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2019/084176**

| Patent document cited in search report | Publication date (day/month/year) | Patent family member(s) | Publication date (day/month/year) |
|---|---|---|---|
| CN 108546731 A | 18 September 2018 | None | |
| CN 107951879 A | 24 April 2018 | None | |
| CN 103429757 A | 04 December 2013 | AU 2012220291 A1 | 03 October 2013 |
| | | US 2014315732 A1 | 23 October 2014 |
| | | EP 2678447 A1 | 01 January 2014 |
| | | CA 2828122 A1 | 30 August 2012 |
| | | RU 2013141965 A | 27 March 2015 |
| | | WO 2012113061 A1 | 30 August 2012 |
| | | JP 2014512515 A | 22 May 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7229619 B


### Non-patent literature cited in the description


- **RICH ; MYSZKA.** *Curr. Opin. Biotechnol,* 2000, vol. 11, 54
- **ENGLEBIENNE.** *Analyst.,* 1998, vol. 123, 1599
- Fundamental Immunology. Raven Press, 1989, 332-336
- Biacore AB, Upsala, Sweden and GE Healthcare Life Sciences. BiaCore. 2000
- **MALMQVIST.** *Biochem. Soc. Trans.,* 2000, vol. 27, 335
- REMINGTON'S PHARMACEUTICAL SCIENCES. Mack Publishing Company, 1995
- Burger's Medicinal Chemistry and Drug Discovery. 1995, vol. 172-178, 949-982
- **J. SAMBROOK et al.** Molecular Cloning A Laboratory Manual. Scientific Press